# EUROPEAN PATENT SPECIFICATION

(11) **EP 0 480 014 B1**
(45) Date of publication and mention of the grant of the patent: **13.11.1996**
(21) Application number: 91909070.4
(22) Date of filing: 16.04.1991
(51) Int. Cl.: A61K 35/14, A61K 39/42, A61K 39/00

(54) **SYNTHETIC POLY-Ig RECEPTOR, RECEPTOR-ANTIBODY COMPLEXES, PRODUCTION AND USE THEREOF**
SYNTHETISCHER POLY-IG-REZEPTOR, REZEPTOR-ANTIKÖRPER-KOMPLEXE, HERSTELLUNG UND ANWENDUNG
RECEPTEUR POLY-Ig SYNTHETIQUE, COMPLEXES RECEPTEURS-ANTICORPS, PRODUCTION ET UTILISATION

(30) Priority: 16.04.1990 US 510161
(43) Date of publication of application: 15.04.1992
(73) Proprietor: THE PRESIDENT AND FELLOWS OF HARVARD COLLEGE, Cambridge, MA 02138 (US); INSTITUT SUISSE DE RECHERCHES EXPERIMENTALES SUR LE CANCER, CH-1066 Epalinges (CH)
(72) Inventor: KRAEHENBUHL, Jean-Pierre, CH-1812 Rivax (CH); WELTZIN, Richard, A., Wellesley, MA 02181 (US); NEUTRA, Marian, R., Sherborn, MA 01770 (US)
(74) Representative: Pidgeon, Robert John
(86) International application number: US9102604
(87) International publication number: WO9116061

(56) References cited:
- THE JOURNAL OF CELL BIOLOGY, vol. 110, no. 4, April 1990, pages 987-998, New York, US; E. SCHAERER et al.: "Polarized transport of the polymeric immunoglobulin receptor in transfected rabbit mammary epithelial cells"
- Biochemical Journal, Vol. 257, issued 1989, SOLARI et al, "Cellular location of the cleavage event of the polymeric immunoglobulin receptor and fate of its anchoring domain in the rat hepatocyte" pages 759-68.
- Journal of Biological Chemistry, Vol. 263, No. 9, issued 25 March 1989 ELIASSON et al, "Chimeric IgG-binding Receptors Engineered from Staphylcoccal Protein A and Streptococcal Protein G". Pages 4323-27. See entire document.
- CRAGO et al., The Journal of Immunology, Vol. 142, Nol. 11, issued 01 June 1989, "Antisera to the secretary component recognize the murine FC Receptor for IgA", pages 3909-12, see Abstract.
- Advances in Experimental Medicine and Biology, KRAEHENBUL, et al., Vol. 216B, issued 1987, "Receptor mediated transepithelial traugrot of secretory antibodies and engineering of mucosal antibodies", pages 1053-60. See Abstract.
- Advances in Experimental Medicine and Biology 216B, 1053-1060 (1987)

## Description

### Background of the Invention

This invention was made in part with funding from the United States Government, and the U.S. Government has certain rights in the invention.

This invention relates to the general fields of passive mucosal immune protection, and of poly-Ig immune reagents and techniques. We use the term poly-Ig to refer to the polymeric classes of antibodies--i.e. IgA and IgM. IgM antibodies are generally produced at an early stage of the immune response and are not an important factor in protective mucosal immunity. Thus, the invention generally refers to polymeric Ig antibodies, and the usual and preferred antibodies for all aspects of the invention are IgA class antibodies, which normally are secreted in dimeric form and, to a lesser degree, as higher IgA polymers.

Many pathogenic bacteria and viruses initially gain entry into the body by crossing the cellular linings (epithelia) of the gastrointestinal, respiratory, or genital tracts. A specialized class of antibodies, IgA antibodies, protects these surfaces. IgA antibodies are dimeric or polymeric molecules produced by cells located in the tissues under the epithelial surfaces. They are transported by epithelial cells into mucosal secretions, where they cross-link or coat pathogens that have not yet entered the body, preventing the pathogens from contacting and adhering to epithelial cells. Thus, IgA antibodies operate on pathogens that are outside the body, and they protect by preventing entry into the body across epithelial surfaces.

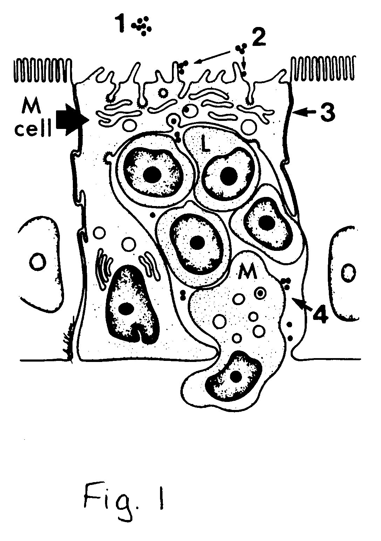

The naturally occuring IgA response is triggered by antigen delivery to mucosal surfaces. The antigen enters the body through specific sampling sites (termed microfold or M cells) that effect transepithelial antigen transport to areas of the mucosal lining containing specialized, organized collections of the cells of the mucosal immune system. More specifically, as shown in Fig. 1, antigens A (shown as filled dots) in lumen 1 bind the luminal surface of M cells at site 2. The antigens are internalized and transcytosed at 3 to be released in the intra-epithelial pocket 4 containing lymphoid cells L (B and T cells) and antigen-processing/presenting cells such as macrophage cells (M).

IgA antibodies in a naturally immunized host are transported into secretions by binding to a specific receptor (called the poly-Ig receptor) on the basal (interior) surfaces of epithelial and glandular cells throughout the respiratory and digestive systems, the genital tract, and the mammary glands. See Solari and Kraehenbuhl, "Receptor-Mediated Transepithelial Transport of Polymeric Immunoglobulins", pp.269-298 in The Mammary Gland, Nelville and Daniel Eds., Plenum Publishing, Cambridge (1987); Mestecky (1987) J. Clin. Immunol. 7:265-276. Receptor-IgA complexes are transported across these cells and exocytosed onto luminal (exterior) cell surfaces where the receptor is enzymatically cleaved, releasing IgA into secretions along with a receptor fragment called secretory component (SC). See Mostov et al. (1980) Proc. Nat'l Acad. Sci. U.S.A. 77:7257-7261; Solari, R. and Kraehenbuhl, J.P. Cell 36:61-71 (1984); Kuhn and Kraehenbuhl, J .Biol. Chem. 256:12490-12495(1981). It is reported that secretory component reduces proteolytic degradation of IgA. Lindh, J., J. Immunol. 114:284-286 (1975); Brown et al. J. Clin. Invest. 49:1374 (1974).

In general, existing immunization strategies which involve injection of antigens evoke production of the IgG class of antibodies that circulate systemically and neutralize pathogens after they have entered the body. Injection of antigens does not generally evoke a substantial IgA response.

Efforts to take advantage of IgA protection at mucosal barriers involve oral immunization, either for active protection of the immunized mammal or for passive protection of another mammal using mucosal secretion of the immunized mammal. Glass et al., New Eng. J. Med., 308:1389-1392 (1983); Fubara et al., J. Immunol., 111(2):395-403 (1973). Monoclonal IgA antibodies have been produced and applied directly to respiratory mucosal surfaces in an effort to protect against pathogen entry. Mazanec et al. J. Virol., 61:2624-2625 (1987).

The generation of poly-IgA producing hybridoma is described in Advances in Experimental Medicine and Biology 216B, 1053-1060 (1987).

### Summary of the Invention

We have discovered various ways to manufacture and stabilize monoclonal poly-Ig antibodies yielding reagents for passive mucosal immune protection. More specifically, those reagents are substantially isolated complexes between poly-Ig specific for a selected antigen or family of antigens, and a stabilizer protein comprising at least one poly-Ig binding domain of the poly-Ig receptor. The poly-Ig stabilizer substantially lacks any C-terminal transmembrane or intracellular poly-Ig receptor domains. The resulting complex is characterized by proteolytic stability in comparison to uncomplexed poly-Ig, and the complex has the specific binding and immunoprotective cross-linking properties of the poly-Ig. In a first aspect of the invention, the stabilizer protein is a recombinant protein. In a second aspect of the invention, the poly-Ig antibody is a monoclonal antibody.

As detailed below, one preferred form of the complex is characterized by covalent (disulfide) bonds between the poly-Ig and the stabilizer. Such complexes can be made by a cell capable of producing the desired poly-Ig specific for the selected antigen or family of antigens, which cell has been engineered to produce poly-Ig stabilizer. The complex is produced by, and recovered from, a culture of such cells.

Preferably, the poly-Ig is a monoclonal IgA class antibody. Also detailed below, the preferred stabilizer can be characterized with reference to the domains of the naturally occurring poly-Ig receptor, and the preferred stabilizer comprises domain I and least domain IV or V, or both, with domain VI being substantially truncated (deleted). Domain I preferably is spaced from domain V by a sequence of about 100-500 amino acids.

A third aspect of the invention features recombinant poly-Ig stabilizer comprising a poly-Ig binding domain of the poly-Ig receptor and substantially lacking C-terminal transmembrane or intracellular poly-Ig receptor domains. Preferred stabilizers are those discussed above regarding the complex featured in the first two aspects of the invention.

A fourth aspect of the invention features methods for assaying protective capacity of poly-Ig monoclonal antibodies in vivo, by introducing the monoclonal poly-Ig to be assayed into the circulation of a mammal, and assaying mucosal secretions of the mammal for the poly-Ig. The mammal is then challenged with the pathogen (or spermatozoa) and protection against the pathogen or insemination is determined. Preferably the monoclonal poly-Ig is introduced into the mammalian circulation by injection or infusion. Alternatively, a tumor or tumor-forming cell (i.e., the hybridoma) can be introduced subcutaneously as a continuous source of the monoclonal poly-Ig.

A fifth aspect of the invention features a method for making the above-described sulfhydryl cross-linked complex by: providing anchorage dependent epithelial cells engineered to produce poly-Ig receptor; culturing those epithelial cells under conditions forming a monolayer of the cells on a porous support that is positioned to separate a first medium on the basal side of the monolayer from a second medium on the apical side of the monolayer. Monoclonal IgA is introduced to the basal side of the monolayer and the complex is formed in the cell as the antibody is transported across the monolayer. The complex is recovered from medium on the apical side of the monolayer. Preferably, the first medium is a nutrient medium suitable for supporting growth of the epithelial cells. Also preferably, the second medium is substantially free from proteins interfering with recovery of the poly Ig complex. The monoclonal IgA may be introduced by co-culturing hybridoma cells in the first medium. Alternatively, the monoclonal IgA is first produced in a hybridoma culture, then separated from the culture, and finally introduced into the first medium.

The complex of the first and second aspects of the invention can be administered directly to mucosal surfaces of a mammal to protect against a pathogen or against insemination. Preferably the complex is administered orally, nasally, vaginally, rectally, ocularly, or to the middle ear. One preferred method of administration is to provide the complex in a mucosal secretion of a first mammal and then to administer that mucosal secretion to a second mammal. For example, milk from the first mammal containing the complex can be fed to the second mammal. In order to generate the secretion, poly-Ig (e.g. purified from hybridoma culture) is introduced into the circulation of the first mammal (e.g. by infusion or injection), and milk or other mucosal secretions are collected from the mammal.

The invention provides particularly effective and safe passive immunization because it is non-systemic. Accordingly, animal (mammalian non-human) immunological reagents are more likely to be tolerated in mucosal immune protection than is true for systemic immunization. Neonatal infections such as necrotizing enterocolitis and Group B streptococcal infections are particularly important targets, given the lack of natural mucosal immunity at this age.

A sixth aspect of the invention features generating a poly-Ig producing hybridoma by challenging a mammal with an antigen and recovering lymphoid cells from mucosal tissue of the mammal such as Peyer's patches or other mucosal tissue rich in lymphoid cells. While Peyer's patches are preferred, other suitable mucosal tissues rich in lymphoid cells include tonsils, adenoids, appendix, and rectal lymphoid tissue. The lymphoid cell is fused to a myeloma cell. Preferably the antigen is applied to a mucosal surface of the mammal.

Other features and advantages of the invention will be apparent from the following description of the preferred embodiments thereof.

### Description of the Preferred Embodiments

### Figures

Fig. 1 is a diagrammatic representation of transcytosis of antigen across M cells.

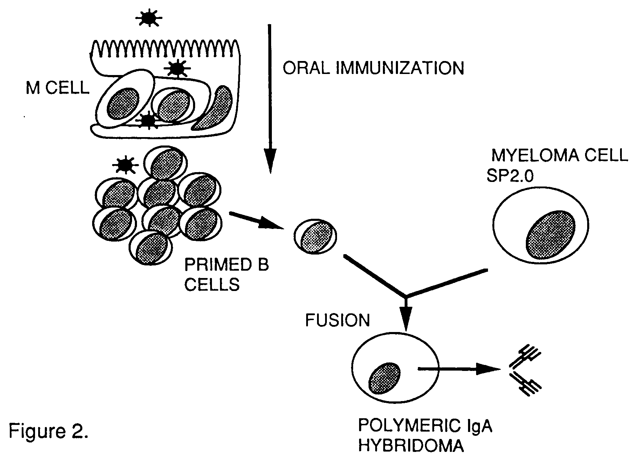

Fig. 2 is a diagrammatic representation of the steps in formation of a polymeric IgA-producing hybridoma.

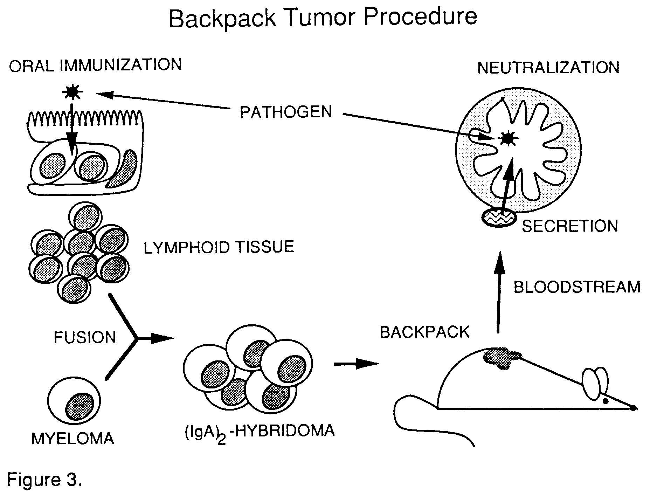

Fig. 3 is a diagrammatic representation of a method for evaluating the protective characteristics of polymeric IgA produced by a hybridoma.

Fig. 4 is a diagrammatic representation of the structure of the complex between polymeric IgA and modified poly-Ig receptor.

Fig. 5 is a diagrammatic representation of the domains of poly-Ig receptor.

Figs. 6A and 6B show the nucleotide sequence of a gene encoding poly-Ig receptor and the deduced amino acid sequence.

Fig. 7 is the deduced amino acid sequence inferred from cloned cDNA's of two rabbit alleles of poly-Ig receptor.

Figs. 8A-8C show the cDNA from which the sequence of Fig. 7 was deduced.

Fig. 9 compares the amino acid sequence of Domain I of the poly-Ig receptor alleles of Fig. 7.

Fig. 10 diagrams the components of various expression vectors.

Fig. 11 diagrams construction of two specific expression vectors, pLK-neo 6.8kb and pLM-neo 6.8kb.

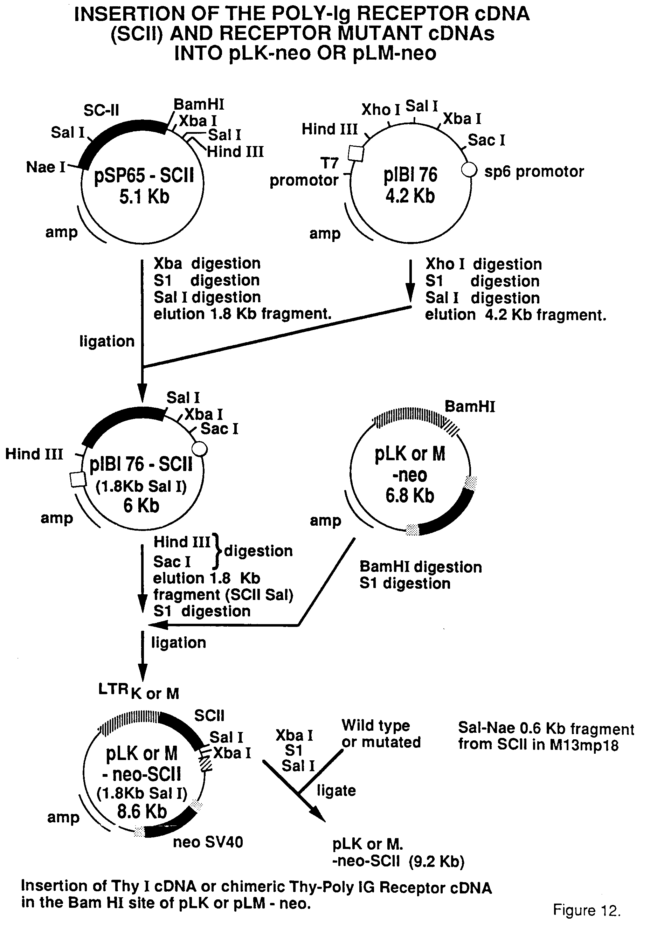

Fig. 12 depicts insertion of the cloned poly-Ig receptor gene into the vectors of Fig. 11.

Fig. 13 depicts various restriction sites in the poly-Ig receptor cDNA.

### Stabilizer Protein and Poly Ig Complex

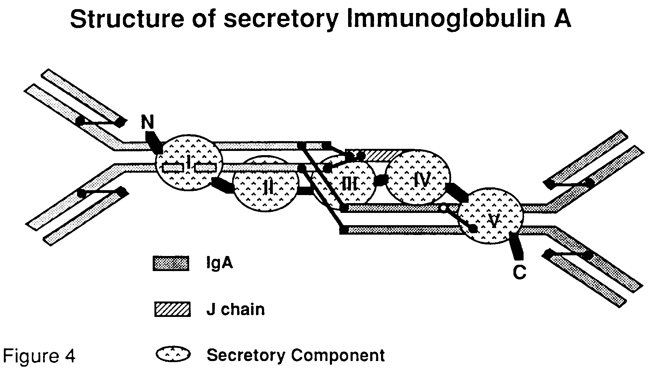

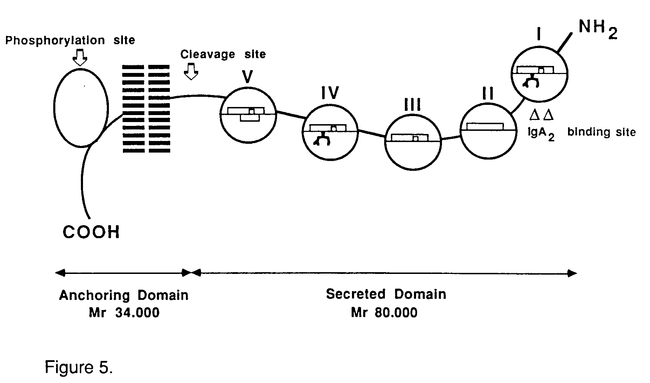

The preferred stabilizer protein is derived from the poly-Ig receptor, a transmembrane molecule which mediates the transport of poly-Ig. The poly-Ig receptor includes the following domains shown in Figs. 4 and 5: five extracellular domains; a transmembrane spanning segment; and a C-terminal intracellular tail. Proteolytic cleavage of the receptor yields the free five-domain protein "SC" or secretory component. Mostov et al. Nature 308: 37-43 (1984); Kuhn et al., J. Biol. Chem., 258:6653-6659 (1983). Disulfide bridges are formed between poly-Ig receptor and IgA, as generally shown in Fig. 4.

The poly-Ig receptor is generally conserved across mammalian species, and we use that term to include all such receptors regardless of the mammalian source, e.g., mouse, rat, rabbit, guinea pig, pig, sheep, horse, cow, or human. Those in the field will appreciate that the rabbit poly-Ig receptor sequences provided in this application may be used in standard hybridization cloning techniques to identify cDNA's encoding poly-Ig receptors from other mammals.

As summarized above, the stabilizer protein portion of the complex can be recombinant stabilizer protein produced in at least two different ways. The poly-Ig receptor can be cloned and expressed in a cell that is capable of exporting it and cleaving it to yield the recombinant stabilizer protein. Alternatively, the poly-Ig receptor gene may be engineered in various ways (particularly by inserting a stop codon as described below) to yield a construction which is directly expressed as the stabilizer protein.

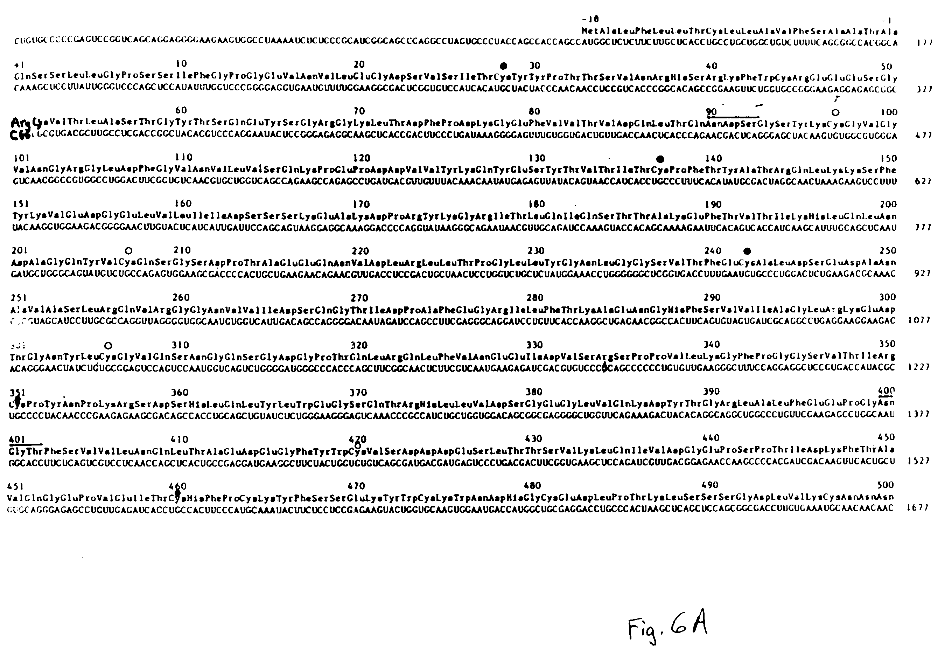

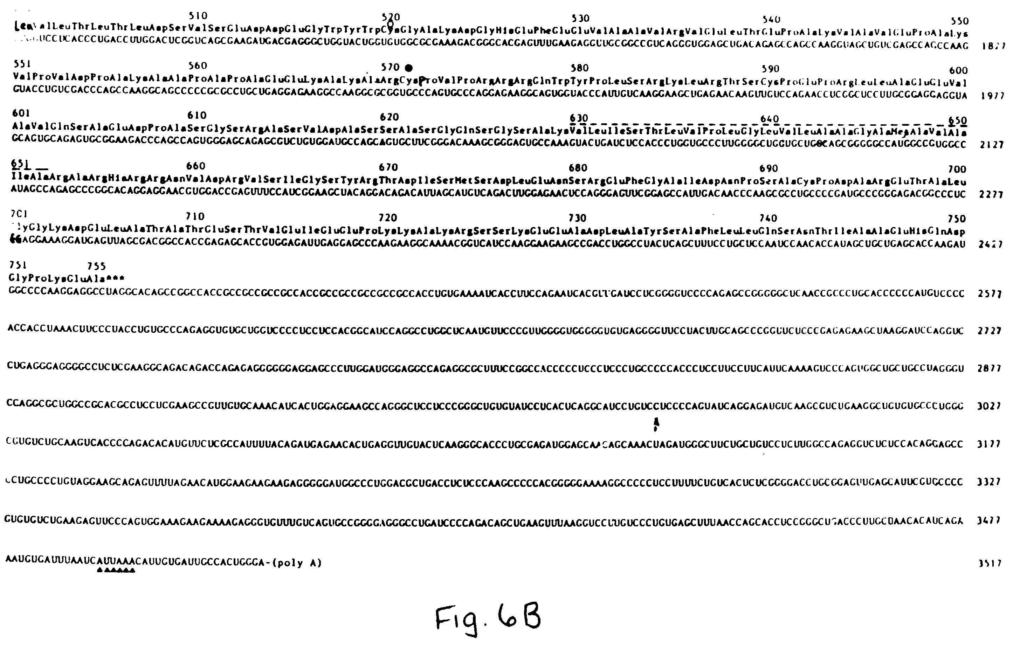

As indicated above, both ways for making the stabilizer protein begin with the cloned poly-Ig receptor gene. Mostov et al., Nature 308:37-43 (1984) report the cloning and sequence of this gene. Fig. 6 is taken from that report and is explained in greater detail below.

Another specific protocol for cloning the gene is as follows. See, Schaerer et al. J. Cell. Biol. 110:987-998 (1990). Cytoplasmic RNA was prepared from the mammary gland of lactating New Zealand rabbits according to Suard et al. Biochem. J. 201:81-90 (1982); and the poly A+ RNA was purified according to Schibler et al. J. Mol. Biol. 142:93-116 (1980). The poly A+ RNA, enriched in receptor translatable activity by size fractionation on a 5-20% linear sucrose gradient, served as a template for the synthesis of cDNA. The first cDNA strand was synthesized according to Wahli et al. Dev. Biol. 67:371-383 (1978) and the second strand according to the DNA polymerase I-RNase H method Gubler, Gene 25:263-269 (1983). The double stranded and dC-tailed cDNA was frantionated on a 1% low melting agarose gel and cDNAs with a size ranging between 1.5 and 7 kb were eluted and annealed to dG-tailed pBR322 as described by Peacock et al., Biochem. Biophys. Acta 655:243-250 (1981). DHl cells were transformed with the annealed DNA by the general method of Hanahan et al., J. Mol. Biol. 166:577-580 (1983). Transformed bacteria were lysed and the DNA transferred onto nitrocellulose filters (Thomas, Methods Enzymol. 100:255-266, 1983) and probed with two synthetic oligonucleotides. Specific probes used were nucleotides 186-206 and 2421-2437 of sequence published by Mostov et al., Nature 308:37-43 (1984), labeled with T4 polynucleotide kinase and αP³²-ATP as described by Gough, N. et al., Nature 309:763-767 (1984). The cDNA clones hybridizing to both probes were further characterized by hybrid selected translation (Riezman et al., EMBO J. 2:2161-2168, 1983). Two resulting plasmids are pSC-l which corresponds to the low MW. form of the poly-Ig receptor with deletion of domains 2 and 3 as described by Deitcher et al., Mol. Cell Biol. 6:2712-2715 (1986); pSC-ll encodes the high MW. form.

Figs. 6A and 6B show the sequence of poly-Ig receptor mRNA and the amino acid sequence predicted from it, reported by Mostov et al. Their characterization of Figs. 6A and 6B is as follows. Residues are numbered from 5' to 3', beginning with the first nucleotide after the poly(dG) tail in p21,53. Nucleotide numbers are indicated at the right margin. The complete sequence is 3,517 bases long and contains the unusual poly(A) addition sequence AUUAAA beginning 26 residues upstream from the poly(A) tail and indicated by triangles under each residue. The predicted amino acid sequence is shown above the nucleotide sequence. The signal peptide is numbered from -18 to -1. The first amino acid of the mature poly-Ig receptor (Gln) is number 1. The two potential sites for Asn-linked glycosylation are overlined. The predicted membrane-spanning region is indicated by a broken overline. Cysteine pairs for the five domains, as well as the corresponding first cysteine for the sixth domain, are indicated by solid circles above the first Cys of each domain and open circles above the second Cys of each domain.

Having cloned the poly-Ig receptor gene, it can be engineered for direct expression of the desired stabilizer protein. Specifically, recombinant proteins containing certain poly-Ig-binding domains of SC will stabilize polymeric Ig against proteolytic cleavage, substantially without detrimental effect on the specific binding and the protective functions of the polymeric IgA. In particular, the domains of Figs. 4 and 5 are the primary poly-Ig binding domain (I), two spacer primary domains II and III and C-terminal domains IV and V which aid poly-Ig stabilization.

The poly-Ig receptor is truncated at the C-terminus to remove the transmembrance and intra-cellular portions of the protein, which are not essential to stabilization or protection. The truncated poly-Ig receptor useful in the invention, therefore, includes (at a minimum) the N-terminus poly-Ig binding domain I.

The following discussion of two specific truncated poly-Ig receptors is provided to illustrate the invention, not to limit it. Those in the field will readily appreciate that the specific protein sequences provided below can be modified with conservative substitutions and deletions that retain the function and spirit of the invention.

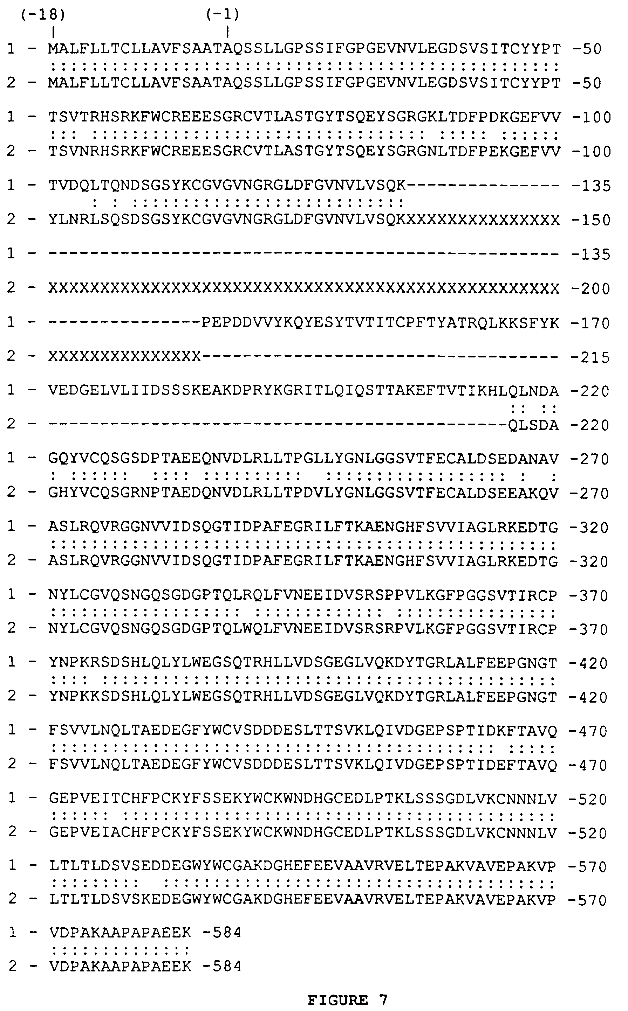

Fig. 7 depicts the amino acid sequence inferred from cloned cDNA's of two rabbit alleles of the poly-Ig receptor. One allele is designated "No. 1" and corresponds to the cDNA reported in Mostov et al., Nature 308:37-43 (1984) shown in Figs. 6A and 6B. The other allele was cloned in the laboratory of applicant Kraehenbuhl, by the technique reported above, and the sequence of that allele is designated "No. 2" in Fig. 7. In Fig. 7, a colon (":") is used to show identical residues; a period (".") is used to show similar residues--i.e., A,S,T; D,E; N,Q; R,K; I,L,M,V; F,V.

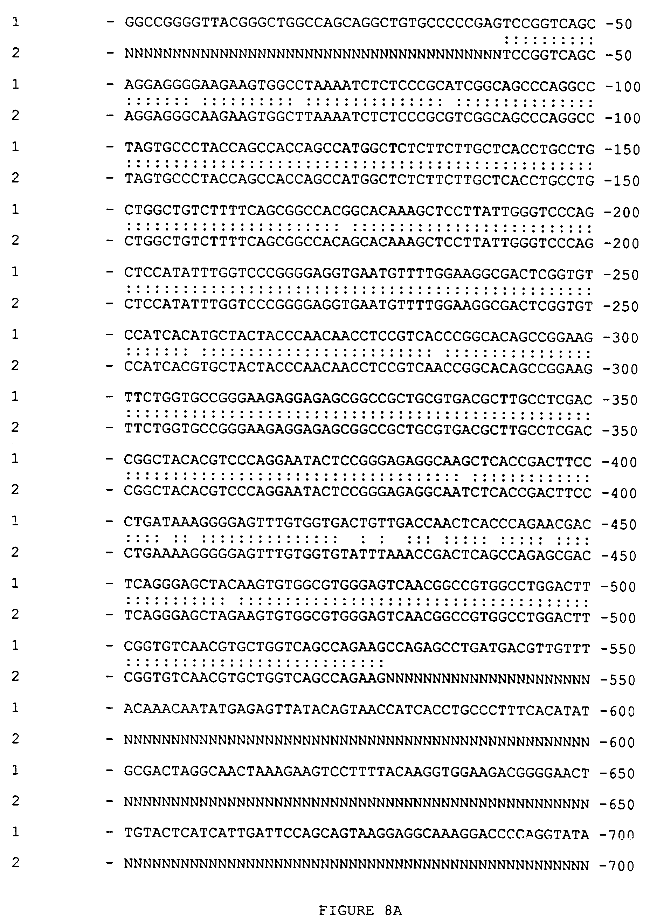

Figs. 8A-8C depict the cDNA sequences of those clones. Again, a colon shows identical bases. "N" shows an unknown base.

The seven domains within the poly-Ig receptor sequence, as follows (the numbers refer to Fig. 6):

| Domain | Segment |
|---|---|
| I | 10-118 |
| II | 119-223 |
| III | 224-332 |
| IV | 333-441 |
| V | 442-552 |
| VI | 553-652 |
| VII | 653-755 |

Domain I is a poly-Ig binding domain, which is retained in the truncated poly-Ig receptor of the invention.

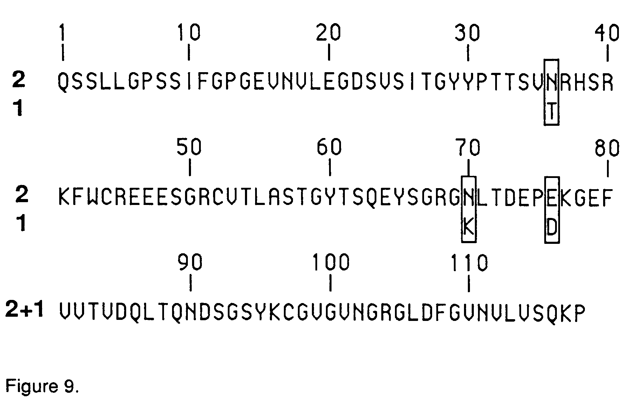

The specific sequence of Domain I for both alleles (including amino acids 1-9 which are not essential for poly-Ig binding and therefore are not critical to this invention) is shown in Fig. 9.

The poly-Ig receptor sequence may be modified by conservative amino acid substitutions, deletions and additions which do not alter poly-Ig binding. Indeed, as shown in Fig. 9, a comparison of the two Domain I sequences shows only three amino acid variations at positions 38, 70, and 76. In each of the other Domains at issue in this invention (II-V), there are between 2 and 6 amino acid changes, Domain IV having changes at residues 337 and 358 and Domain V having changes at residues 448, 460, and 513.

In sum, there is well over 80% homology at the amino acid level, and the sequences can be modified in all five domains by amino acid substitutions or by domain deletions.

In particular, sufficient homology should be retained in domain I for poly-Ig association, proper spacing (e.g. 100-500 amino acids) should be retained between domains I and V, and domain V should be suitable to retain covalent disulfide stabilization. Thus, very significant modification can be tolerated in domains II-IV.

It is highly desirable that the truncated poly-Ig receptor also include domain V which, via a sulfhydryl linkage, is responsible for the covalent stabilization of the complex. See, generally Eiffert et al., Hoppe Seyler's Z. Phys. Chem. 365:1489-1495 (1984) identifying the cysteine residues of poly-IgA (human) involved in formation of disulfide bridges. It is important, in addition, to include a spacer corresponding to domains II-V, to position domain V for disulfide bridge formation as shown in Fig. 5, and to enhance protection against proteolysis.

To determine the appropriate location for truncation, the membrane-spanning segment in domain VI of the complete receptor can be identified as a stretch of uncharged predominantly hydrophobic residues (e.g. 630-652 in Fig. 6) preceded by charged residue (e.g. Lys 629) and followed by an extremely basic sequence (e.g. Arg-Ala-Arg-His-Arg in Fig. 6). This membrane spanning portion, as well as the C-terminal cytoplasmic tail of the molecule, are deleted by the truncation.

In one specific embodiment, truncation was achieved by synthesis of a 16-mer oligonucleotide: ACTAGTCTAGACTAG having the stop codon TAG in all three reading frames. The oligonucleotide is a palindrome and self-hybridizes. The resulting duplex was inserted into the above-described poly-Ig cDNA (DNA position 1834, amino acid 554)

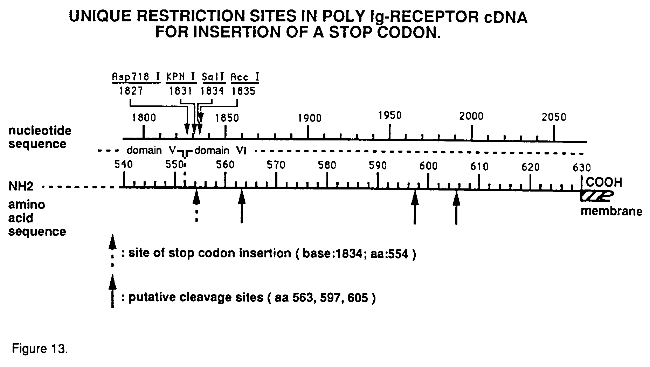

More specifically, the four amino acids (T-S-L-D) encoded by the oligomer are added to the C-terminus of domain V, so that truncation occurs precisely at the transition between domains V and VI. The restriction endonuclease site selected for oligomer insertion is the SalI site that is unique and close to the putative natural cleavage sites (amino acids 563, 597, and 605). Other convenient insertion sites in domain V are at Asp (1827); KpnI (1831); AccI (1835). There are no convenient unique sites in domain VI. See Fig. 13.

The above described cloned poly-Ig receptor cDNA or engineered (e.g. truncated) poly-Ig receptor cDNA may generally be expressed in any of a wide variety of expression systems that are well known to those in the field. For example, desirable expression systems are professional secretory cells (i.e., cells that naturally secrete large amounts of protein) such as fibroblast cells, including Chinese Hamster Ovary expression systems. Endocrine tumor cell expression systems such as ATt 20 cells (ATCC CRL89 and CRL1795) or PC-12 cells can be used. Madin-Darby Canine Kidney (MDCK) cells are also desirable host cells. See Mostov et al. (1984) cited above. The choice of expression vehicle will depend on the host cell that has been selected.

In one embodiment, the truncated poly-Ig receptor may be expressed in immortalized IgA-producing cells, such as lymphocyte-myeloma fusions. In that case, the preferred expression vehicle is one which includes a strong or an inducible promoter, enhancer element (e.g. a B-lymphocyte specific enhancer, such as the µ-enhancer, or an enhancer of viral origin), an engineered stop codon introduced into the poly-Ig R-cDNA to avoid the need for proteolytic separation of domains l-V (SC) from the cell membrane anchor (domain VI-VII). Specific expression systems are described below.

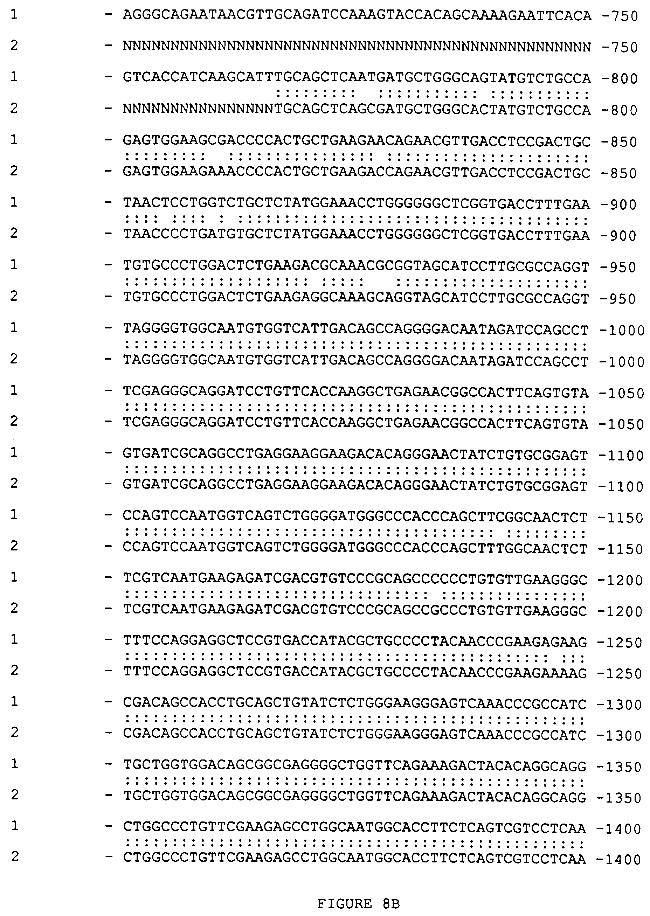

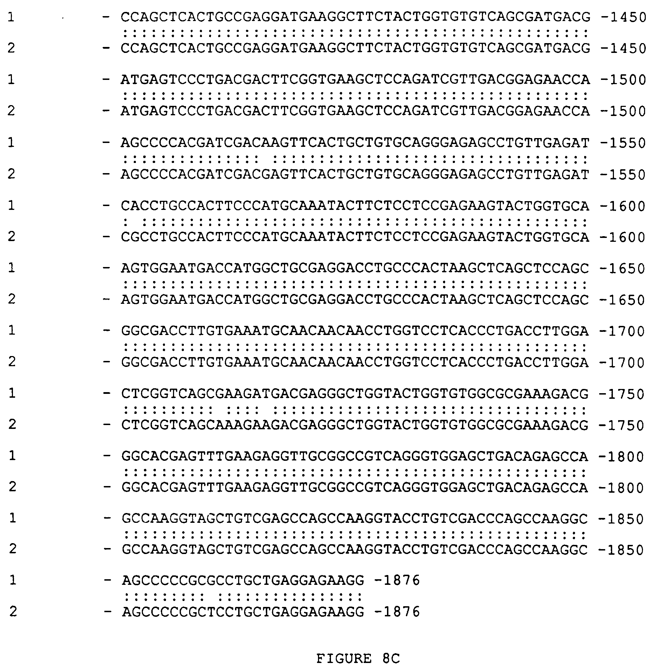

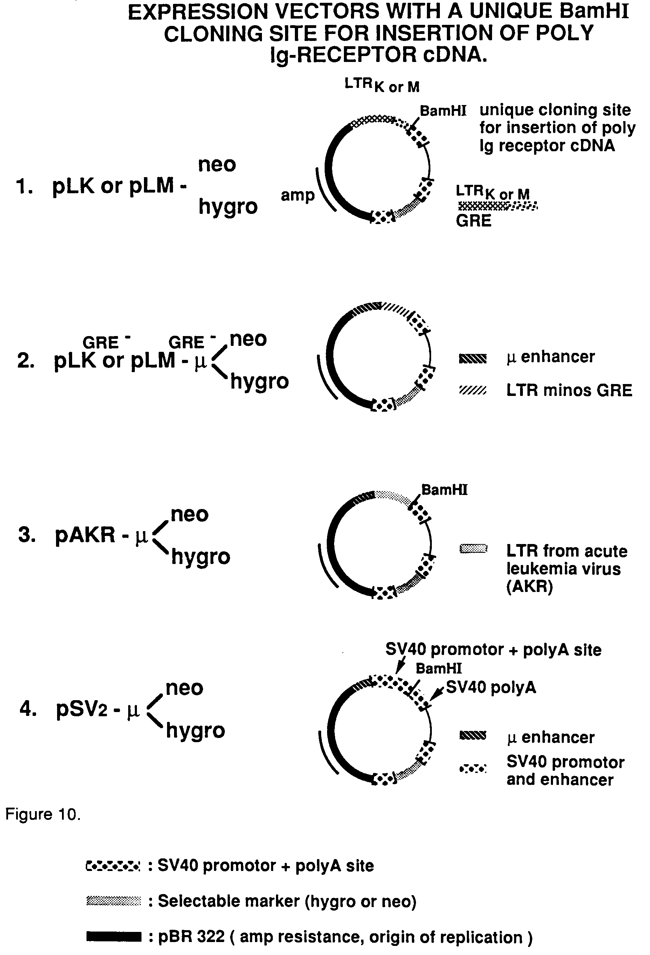

As shown generally in Fig. 10 the expression vehicle may be a vector exemplified by pLK-neo or hygro or the pLM-neo or hygro having glucocorticoid responsive elements or by the same vectors in which the glucocorticoid responsive elements have been removed and replaced by a µ enhancer element, pAKR-µ-neo, and pSV4O-µ neo. The vector may include the following elements.
1) An appropriate promoter for initiating transcription of the cDNA. LTR's from retroviruses are suitable, including the LTR isolated from kidney adenocarcinoma as described by Williger et al., J. Virol. 60:1-11 (1986). The kidney LTR shares homology with the LTR isolated from mammary tumors, with some differences in the U₃ region. Both LTRs have a glucocorticoid responsive element which allows hormone stimulation of transcription. For example, glucocorticoids (dexamethasone, Sigma Corp., St. Louis, MO) increases transcription of truncated poly-Ig receptor by 10-50 fold. The kidney or mammary LTR recovered from a cloning vector (pLK or pLM: provided by Dr. Heidi Diggelmann, Swiss Institute for Cancer Research, in whose group the plasmid was constructed, see Williger et al. cited above) by sequential pStl, S1 and BamH, digestion, was inserted into a pSV₂ neo vector (commercially available through Biolabs), which was previously and sequentially digested with EcoRI, S1 and BamHl digestion (to remove the SV40 promoter and replace it by the LTR promoter). (Fig. 6B). The pLK-hygro or pLM-hygro vector was constructed by introducing the hygromycin resistance gene (1B) in place of the neomycin resistance gene. We have deposited plasmid PLKₙₑₒPIRll with the ATCC, Rockville, Maryland, under accession number ATCC 40787. That plasmid is the pLKₙₑₒ plasmid shown in Fig. 10 with the truncated polymeric immunoglobulin receptor-encoding DNA (Fig. 8) inserted in the BamHI site of the plasmid.
2. Enhancer elements to enhance transcription and hence production of truncated poly-Ig-receptor. Such elements include viral elements (SV40, glucocorticoid responsive elements of MMTV) or mammalian tissue specific enhancers such as the µ enhancer responsible for high expression of Ig in B cells. The µ enhancer recovered from a mouse genomic clone encompassing the µ heavy chain locus (Mercola, M, Wang, X.F., Olsen, Y, and Calame, K. 1983, Science 221:663-665), can be inserted upstream of various promoters.
3. A vector backbone including selectable markers (a neomycine or hygromycine resistance gene for instance), an origin of replication in a E. coli with an antibiotic resistance gene.
4. The poly-Ig-receptor-encoding cDNA.
5. A synthetic oligomer containing stop codons in each reading frame, e.g. ACTAGTCTAGACTAGT.

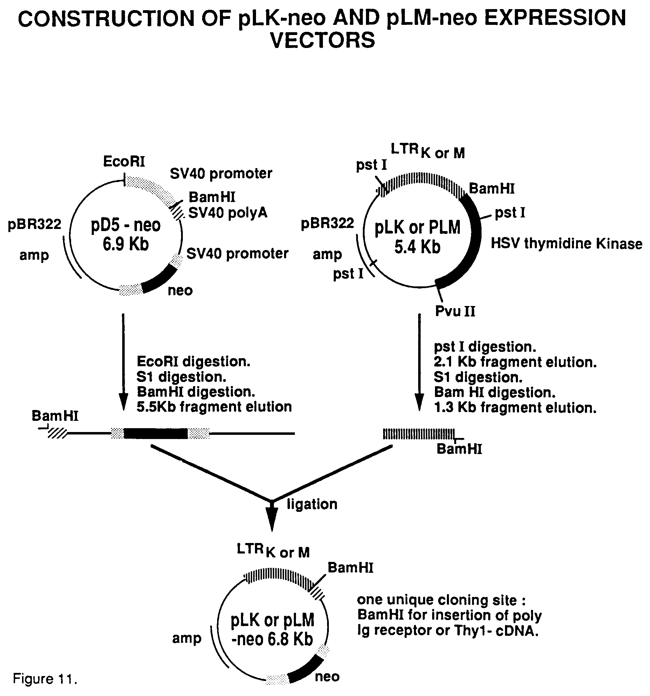

Additional detail for the construction of such vectors is provided in Fig. 11. The cDNA for pSC-l was introduced into the expression vector pSV-2 described by Southern and Berg, J. Mol. Appl. Gen. 1:327-341 (1982). The construct is referred to as pSV2-SCl.

The resulting vector was transfected into myeloma cells and cells were isolated to show efficient expression of truncated poly-Ig receptor. Standard electroporation techniques were used to effect the transfection.

Specifically, cells are harvested from growth medium (2x10⁶ per sample) and spun down (5'x1000rpm). The cells should come out of cultures in a 30-70% confluency state and the medium should have been changed in the previous 24 hours. At chilled temperature (ice bath) the cells are washed with electroporation medium (RPMI 2mM glutamine and 0.5% fetal calf serum with Pen Strp®).

The cells are counted and resuspended in the electroporation medium at 2x10⁶/0.75ml. About 45-50µl of linearized DNA (10µg linearized and resuspended in 45µl phosphate-buffered saline) is added to an aliquot (0.75ml) of the cell suspension in an electroporation cuvette. The cuvette is allowed to sit on ice for 15 minutes, and the mixture is pulsed. Cells are then plated in conditioned RPMl or Eagle's medium and selected.

The transfected myeloma cells express and secrete the truncated poly-Ig receptor. In contrast, myeloma cells are unable to cleave and secrete the full receptor. The truncated poly-Ig receptor is isolated and purified from the myeloma culture supernatant by ammonium sulfate precipitation and gel filtration.

In one particularly preferred embodiment described in greater detail below, the truncated poly-Ig receptor is produced in a hybridoma which has been selected for its production of a desired poly-IgA.

In such a system, the covalent disulfide bonds linking the truncated receptor to the poly-Ig are formed in the living cells and the resulting stable secretory Ig complex is secreted. This system can be constructed either by transfecting myeloma cells that will be used as fusion partners to immortalize poly-Ig-producing B cells, or by transfecting hybridomas that have already been created and screened for production of the desired poly-Ig. In either case, the resulting covalent disulfide linkage is produced.

Transfection of SP-20 or p 3 x 63/Ag 8U.l myeloma cells (ATCC CRL1581, CRL8287, CRL1597) was carried out using a pLK expression vector in which the poly-Ig receptor cDNA transcription is under the control of the kidney MMTV LTR promoter. Williger et al. J. Virology 60:1-11 (1986). Efficient transcription is obtained by stimulating the transfected cells with 10⁻⁶ M dexamethasone for at least 12 hours.

Another system for producing the stabilizer protein involves transfecting an expression vehicle for expressing the complete poly Ig receptor into a mammalian epithelial anchorage-dependent cell line which forms a monolayer on a porous substrate. One particular such cell line is the MDCK cell line transformed with a vector for expressing the poly-Ig receptor as described above. Specifically, the pLK-neo-ScII vector containing the glucocorticoid LTR_{K} can be used. Other suitable monolayer-forming epithelial host cells may be used. The hygromicin resistance gene is available as described by Bloechliner and Diggelmann, Mol. Cell. Biol. 4:2929-2931 (1984).

In general, the transfected cell line is grown to a monolayer on a porous substrate, such as a filter (e.g. transwell filters, 0.45µM, 4.7cm²) coated with collagen. See Steele et al., Am. J. Physiol. 251:C136-C139 (1986). The cells will become asymmetric, having basal and apical membranes. The membrane and monolayer are arranged to separate a vessel into a basal medium and an apical medium. Since the cells tend to feed from the basal medium, that medium should have the nutrients necessary to maintain cell growth. There is less demand for nutrients in the apical medium.

Advantageously, the cells will use the recombinant poly-Ig receptor to take up poly-Ig from the basal medium and to transport it to the apical membrane where the receptor is enzymatically cleaved, yielding stabilized poly-IgA covalently associated with the stabilizing protein. The poly-IgA is supplied to the basal medium by culturing the IgA-producing hybridoma in that medium. Alternatively, the poly-Ig recovered (e.g. by affinity purification) from a separate hybridoma culture may be added to the medium.

The stabilized poly-IgA may be recovered from the apical medium, again by affinity purification or other standard antibody separation methods. The apical medium can be maintained essentially free from protein contaminants to simplify purification.

### Construction And Screening of IgA-Producing Hybridomas

Another aspect of the invention is the ability to readily form poly-Ig antibodies and screen for poly-Ig (especially IgA) antibodies affording protection against selected infections, particularly infections by viral and bacterial pathogens that gain entry to the body via mucosal surfaces. This aspect of the invention also includes the ability to form poly-Ig antibodies against surface components of spermatozoa, and to screen for antibodies that afford protection against pregnancy. It further includes formation and screening of poly-Ig antibodies against food and airborne allergens.

In this aspect of the invention, a controlled supply of poly Ig antibodies is provided to a host animal; for example, poly-Ig-secreting hybridomas are implanted in a host animal, where they develop into a tumor. The poly-Ig produced and released by that tumor enters the circulatory system and circulating dimeric monoclonal IgA passes out of leaky capillaries into mucosal tissue, where it is recognized and bound by epithelial receptors, and transported into secretions with the addition of endogenous secretory component. Peritoneal (ascites) tumors of hybridoma cells (which are known) may cause undesirable pathological changes in the intestine and abnormally high levels of non-secretory IgA in the peritoneal fluid bathing the intestine. Accordingly, a preferable site for Ig-A-secreting hybridoma tumors is subcutaneous--e.g. on the upper back.

The IgA-producing hybridoma is created by immortalizing an antigen-sensitized B lymphocyte, e.g. harvested from Peyer's patches (PP) after oral or mucosal immunization. Weltzin et al. J. Cell Biol. 108:1673-1685 (1989). The myeloma cells used to immortalize the IgA producing cell are widely available. Standard fusion protocols can be used.

Specifically, hybridomas are generated and tested for specific IgA production according to the following specific examples.

Various standard methods may be used to challenge animals to produce antigen-sensitized B cells for hybridoma construction. See Weltzin et al. cited above. One particular method involves the use of hydroxyapatite (HA) microparticles as a carrier for orally administered antigen.

HA micro crystals suitable for transport across epithelium (e.g. generally less than about 0.1µm) are coated to saturation with antigen and are compounded in a suitable vehicle for mucosal administration (e.g. a salt solution). For oral immunization of a 25-gram mouse\ a small amount (eNgN, 30 µg) of protein in 200 µl PBS is mixed with an appropriate amount (e.g., 1 mg of HA for 30 µg protein) of pre-sonicated and washed HA. The mixture is agitated for 1 hr at 4°C, and HA is then pelleted by spinning for 2 min at 10,000 rpm in a microfuge. The pellet is washed and vortexed or sonicated to resuspend in 200 µl of water containing 0.1 M (or less) NAHCO₃ (sodium bicarbonate, to neutralize gastric acid.) Administration solution for oral immunization can be adjusted to avoid high salt conditions which tend to release the protein from the HA.

Other suitable means for challenging a mammal to raise a mucosal immune response are known to those in the art and are suitable for use in the invention.

In one specific method for producing IgA-secreting hybridomas, PP mononuclear cells are isolated from a challenged animal. Specifically the mucosa and submucosa of 8-10 PPs were removed and incubated in complete RMPI medium containing 0.1% collagenase for 1 h at 37°C. Tissues were ground between the ends of two sterile frosted-end microscope slides, and released cells were washed two times in complete RPMI. Approximately 2-3 X 10⁷ viable PP mononuclear leukocytes were recovered from one mouse.

Myeloma cells and mouse mononuclear cells were washed twice with serum-free RMPI and fused in 37% polyethylene glycol 4000 (E. Merck, Darmstadt, FRG) at a leukocyte to myeloma cell ratio of 5:1. After fusion, cells were diluted to 1 X 10⁶ cells/ml in complete RPMI and distributed into 96-well tissue culture plates that had been seeded 24 h earlier with 2 X 10⁴ irradiated (800 rads) mouse peritoneal exudate cells per well. Hybridomas were selected in hypoxanthine, aminopterin, and thymidine medium using standard methods.

Hybridomas that produce IgA antibodies directed against the desired antigens or cells are identified using standard screening methods.

The resulting hybridoma cells are grown, e.g., in RMPl 1640 culture medium pelleted, resuspended in phosphate-buffered saline, and counted. Cell density is adjusted to 10⁶ cells per 0.5 ml. The suspended cells (10⁶ in 0.1 to 0.5ml) are injected subcutaneously on the upper backs of BALB-C mice. Firm nodules appear between one and three weeks after injection. Serum samples are taken by tail bleed when the nodules reach an estimated diameter of 3-5 mm. An ELISA is used to measure serum IgA. The secretory IgA of the same specificity as the implanted hybridoma is measured in intestinal washings and in milk, also using an ELISA. Other mucosal secretions (e.g. from epithelia and glands of the oral/nasal cavity, respiratory and genital systems) also presumably contain the specific secretory IgA.

These tumor-bearing mice are then used in "challenge" experiments in which the viral or bacterial pathogen at issue is introduced orally or onto other mucosal surfaces, or in which the semen or spermatozoa are introduced vaginally. Hybridomas secreting useful IgA's are identified from the mice that exhibit protection against the challenge. Such tests can be used to specifically identify secretory IgA's that are most suitable for passive protection or contraception in human and other species (see below). For example, in identifying suitable anti-V. cholerae secreting hybridomas, neonatal mice are implanted at day of birth, in order to conduct the screening before the neonates automatically develop resistance against cholera.

The above test can be adapted for neonatal protection experiments in which the hybridomas are implanted in pregnant BALB/C mice during the first or second week of the 3-week gestation period. Tumor growth occurs during pregnancy, and circulating dimeric IgA from the tumor is efficiently transported by mammary gland epithelial cells and released as secretory IgA into colostrum and milk. The neonates ingest SIgA from birth. Where it is desirable to administer the IgA for a sustained period, multiple lactating females can be used.

Once IgA monoclonal antibodies have been identified as protective antibodies using the in vivo assay described above, they are tested for their ability to protect after direct or passive application to mucosal surfaces. In these tests, poly-Ig with or without stabilizer protein can be produced in culture (as described above) and applied to mucosal surfaces of the organ systems listed previously.

A particularly important feature of this aspect of the invention is the operation of IgA outside the recipient's body, on mucosal surfaces. Thus, the IgA need not be immunologically compatible with the recipient's system; it need only reach the recipient's mucosal surfaces and effectively block entry of pathogens.

The invention is particularly useful to protect against Pseudomonas aeruginosa, Hemophilus influenzae, Vibrio cholerae, Bordetella pertussis, Corynebacterium diphtheriae, Escherichia coli, Salmonella typhi and typhimurium, Clostridium perfringens and other enteric clostridiae, Shigella dysenteriae, Shigella flexnerii Neisseria gonorrheae, Trichomonas, Entameba histolytica, Giardia lamblia, Streptococcus, respiratory syncytial virus, rotavirus, reovirus, Human Immunodeficiency Virus, Human T-Cell Lymphotrophic Virus, Types I and II, polio virus, Rhinovirus, influenza virus, herpes viruses, human papilloma virus; AIDS 2° pathogens such as Pneumocystis, and yeast such as monilia. The invention is also useful to protect against allergens that contact the respiratory or digestive mucosal surfaces. It is also useful to protect against pregnancy by cross-linking spermatozoa in the vagina, and preventing their movement through the cervix and uterus.

In each case, an appropriate known antigen--e.g. whole pathogen or specific externally presented antigens such as the viral coat protein, or bacterial cell-surface proteins, pilus protein, lipopolysaccharides, viral capsid or envelope protein, protozoal plasma membrane surface component, spermatozoal surface proteins, or respiratory allergens--are used. Toxoids, e.g. CRM-197, and inactivated diphtheria toxin reported by Uchida et al. J. Biol. Chem. 248:3838-3844 (1973) may be used. The antigen is used according to the above procedure to generate hybridomas secreting the desired protective antibodies. As just a few examples, WO88/08437 discloses a tcPA pilus protein suitable for forming anti-V. cholerae monoclonal poly-Ig antibodies. U.S. Pat. 4,725,669 discloses the HIV (HTLV-III) envelope glycoprotein suitable for forming anti-HIV poly-Ig monoclonal antibodies. The following patents and patent applications disclose preparation of antigens for protection against Streptococcal infections, particularly infection by Group B Streptococcus: U.S. Pat. 4,367,221; 4,367,222; 4,367,223; 4,356,263, 4,207,414 (RE 31,672); and WO 87/06267.

One suitable IgA monoclonal antibody against V. cholerae lipopolysaccharide is produced by hybridoma 2D6, depositied with the ATCC, Rockville, MD, under accession number ATCC HB10420.

Other suitable antigens are disclosed in Bacterial Vaccines and Local Immunity Proceedings of the Sclavo International Conf., Siena, Italy 17-19 November 1986. Specific reagents suitable for HIV antigens are disclosed in AIDS Research and Reference Reagent Program, National Institute of Health, June 1989. For example, gp 120 is sold by MicroGeneSys, Inc. Spermatozoa cell-surface antigens such as LDH-C4 are also known. See, e.g. Shaha et al. and Talwar et al. Vaccine 7:97-100 (1989); and Shaha et al. Int. J. Androl. 11:479 (1988).

Of particular significance in the selection of antigens for practice of the invention is that mucosal protection involves cross-linking to prevent entry into the body, and this mechanism does not require that the polymeric antibody kill or "neutralize" the pathogen. In contrast, systemic (IgG) protection involves binding which, to be effective, generally must neutralize the pathogen. Thus, not every IgG antibody which binds to the pathogen is protective, as illustrated by the existence of monoclonal antibodies that specifically bind Vibrio cholerae, but do not neutralize it in the sense of preventing it from colonizing, growing and manifesting clinical symptoms in its host. The universe of antigens and determinants available to raise protective IgA antibodies is thus significantly increased.

### PASSIVE IMMUNE PROTECTION

Hybridomas identified by the above method as secreting protective poly-Ig monoclonal antibodies are cultured by known techniques and the antibody is separated, e.g., by specific immunoabsorption, and recovered.

The truncated Ig receptor complex is provided either by transforming the hybridoma with an expression vector as described above or by expressing the Ig receptor separately and adding it to the monoclonal antibody by the monolayer culture technique described above.

The resulting stabilized monoclonal antibody is formulated into a "passive" vaccine that is administered directly to a mucosal surface likely to be challenged by the pathogen at issue.

The specific vehicle and route of administration of the complex will vary depending on the pathogen. Oral administration generally is accomplished by buffered salt solution or encapsulation for protection of the complex against gastric and pancreatic proteases, when desired. Nasal adminstration is accomplished with an aerosol. Ocular administration is accomplished with a saline solution. Genital or rectal administration are accomplished with gels, foams or suppositories.

Those skilled in the art will understand how to formulate such vehicles by known techniques.

### ACTIVE VACCINATION

Hydroxyapatite-conjugated antigens such as those described above for challenging donors of hybridoma fusion partner cells can also be used in active vaccination. Specifically, the conjugated antigen is applied directly to mucosal tissue either orally (using encapsulation or other delivery vehicle) nasally (by aerosol), rectally or vaginally (by suppository or other vehicle).

### Deposits

Applicants' assignee, the President and Fellows of Harvard College, represent that the Deposits referenced above, ATCC 40787 and HB10420 were made under the terms of the Budapest Treaty with the American Type Culture Collection (ATCC) in Rockville, Maryland. They further represent that the ATCC is a depository affording permanence of the deposit and ready accessibility thereto by the public if a patent is granted. All restrictions on the availability to the public of the material so deposited will be irrevocably removed upon the granting of a patent. The material will be available during the pendency of the patent application to one determined by the Commissioner to be entitled thereto under 37 C.F.R. 1.15 and 35 U.S.C. 122. The deposited material will be maintained for a period of at least five years after the most recent request for the furnishing of a sample of the deposited microorganism, and in any case, for a period of at least thirty (30) years after the date of deposit or for the enforceable life of the patent, whichever period is longer. Applicants' assignee acknowledges its duty to replace the deposit should the depository be unable to furnish a sample when requested due to the condition of the deposit.

Other embodiments are within the following claims.

## Claims

1. A substantially isolated complex comprising
a) poly-Ig specific for a selected antigen or family of antigens; and
b) a recombinant stabilizer protein comprising a poly-Ig binding domain of poly-Ig receptor, said stabilizer protein substantially lacking any C-terminal transmembrane and intracellular poly-Ig receptor domains;
said complex being characterized by proteolytic stability in comparison to uncomplexed poly-Ig, said complex being further characterized by the specific binding and immunoprotective cross-linking properties of the poly-Ig.

2. A substantially isolated complex comprising
a) a monoclonal poly-Ig specific for a selected antigen or family of antigens, and
b) a stabilizer protein comprising a poly-Ig-binding domain of poly-Ig receptor, said stabilizer protein substantially lacking any C-terminal transmembrane and intracellular poly-Ig receptor domains,
said complex being characterized by proteolytic stability in comparison to uncomplexed poly-Ig, said complex being further characterized by the specific binding and immunoprotective cross-linking properties of the poly-Ig.

3. The substantially isolated complex of claim 2 in which said stabilizer protein is a recombinant protein.

4. The substantially isolated complex of claim 1, 2, or 3 in which said poly-Ig and said stabilizer protein are covalently joined by disulfide bonds.

5. The substantially isolated complex of claim 1 or claim 2 in which said poly-Ig is poly-IgA class antibody.

6. The substantially isolated complex of claim 1 or claim 2 in which said stabilizer protein comprises domain I and at least one of domains IV and V of poly-Ig receptor, and said poly-Ig stabilizer is truncated to substantially delete domain VI.

7. The substantially isolated complex of claim 6 in which said stabilizer protein is further characterized in that said domain I is spaced from said domain IV or V a distance corresponding to a sequence of about 100 to 500 amino acids.

8. The complex of claim 1 or claim 2 comprising a monoclonal IgA antibody specific for an antigen selected from the group consisting of antigens of Pseudomonas aeruginosa, Hemonhilus influenzae, Vibrio cholerae, Bordetella pertussis, Corynebacterium diphtheriae, Escherichia coli, Salmonella typhi and typhimurium, Clostridium perfringens and other enteric clostridiae, Shigella dysenteriae, Shigella flexnerii Neisseria gonorrheae, Trichomonas, Entameba histolytica, Giardia lamblia, Streptococcus, respiratory syncytial virus, rotavirus, reovirus, Human Immunodeficiency Virus, Human T-Cell Lymphotrophic Virus, Types I and II, polio virus, Rhinovirus, influenza virus, herpes viruses, human papilloma virus; Pneumocystis; yeast; and spermatoza antigens.

9. Recombinant poly-Ig stabilizer protein comprising a poly-Ig binding domain of poly-Ig receptor, said stabilizer protein substantially lacking any C-terminal transmembrane and intracellular poly-Ig receptor domains, said stabilizer protein having the characteristic of forming a complex with poly-Ig that is proteolytically stable and has the immunoprotective cross-linking properties of the poly-Ig.

10. A method for making the complex of claim 4 comprising:
a) providing a cell engineered to produce said stabilizer protein, said cell also being capable of producing a monoclonal poly-Ig specific for said selected antigen or family of antigens;
b) culturing said cell to produce said complex; and
c) recovering said complex.

11. A method for making the complex of claim 2 in which said poly-Ig and said stabilizer are covalently joined by disulfide bonds, comprising
providing anchorage dependent epithelial cells engineered to produce poly-Ig receptor,
culturing said epithelial cells under conditions forming a monolayer of said cells on a porous support, said porous support and monolayer forming separating a first medium on the basal side of the monolayer from a second medium on the apical side of the monolayer,
introducing monoclonal IgA to the basal side or the monolayer
recovering said complex from the apical side of the monolayer.

12. The method of claim 11 in which said first medium is nutrient medium suitable for supporting growth of said epithelial cells.

13. The method of claim 11 in which said second medium is substantially free from proteins interfering with recovery of said complex.

14. The method of claim 11 in which said monoclonal IgA is introduced into said first medium by co-culturing hybridoma cells in said first medium.

15. The method of claim 11 in which said monoclonal IgA is first produced in a hybridoma culture, then separated from said culture, and thereafter introduced into said first medium.

16. A method for screening monoclonal poly-Ig to determine protection against a pathogen or against pregnancy, in vivo, said method comprising the steps of
a) introducing said monoclonal poly-Ig into the circulation of a mammal susceptible to the pathogen;
b) assaying mucosal secretions of said mammal for poly-Ig;
c) challenging a mucosal surface of said mammal with said pathogens or spermatoza;
d) determining protection against the pathogen or spermatozoal challenge.

17. The method of claim 16 in which the monoclonal poly-Ig is introduced into the mammal by injection or infusion into the circulation.

18. The method of claim 16 in which the monoclonal poly-Ig is introduced into the mammal by subcutaneous implant of a tumor or a tumor-forming cell that produces the monoclonal poly-Ig.

19. A composition comprising a complex of any one of claims 1-3 for therapeutic administration to a mucosal surface.

20. The composition of claim 19 for therapeutic administration of the complex orally, nasally, vaginally, rectally, ocularity, or into the middle ear.

21. The composition of claim 19 characterized in that the composition is a mucosal secretion of a first mammal comprising said complex, said mucosal secretion being intended for administration to a second mammal to be protected.

22. The composition of claim 21 characterized in that the composition is milk of said first mammal, for oral administration of said complex to the second mammal.

23. The composition of claim 21 or 22 obtainable by
a) administration to the first mammal of an immortalized cell producing poly-Ig so as to produce said poly-Ig of said complex in the circulation of the first mammal, and
b) collecting from the first mammal the mucosal secretion comprising said complex, the mucosal secretion being for administration of said complex to the second mammal.

## Patentansprüche

1. Im wesentlichen isolierter Komplex, umfassend
a) Poly-Ig, welches spezifisch für ein ausgewähltes Antigen oder eine Antigenfamilie ist; und
b) ein rekombinantes Stabilisierungsprotein, umfassend eine Poly-Ig-Bindungsdomäne des Poly-Ig-Rezeptors, wobei dem Stabilisierungsprotein im wesentlichen jegliche C-terminalen Transmembran- und intrazellulären Poly-Ig-Rezeptordomänen fehlen;
wobei der Komplex gekennzeichnet ist durch proteolytische Stabilität im Vergleich zu nicht-komplexiertem Poly-Ig und wobei der Komplex weiterhin gekennzeichnet ist durch die spezifischen Bindungs- und immunprotektiven Vernetzungseigenschaften von Poly-Ig.

2. Im wesentlichen isolierter Komplex, umfassend
a) monoklonales Poly-Ig, welches für ein ausgewähltes Antigen oder eine Antigenfamilie spezifisch ist und
b) ein Stabilisierungsprotein, umfassend eine Poly-Ig-Bindungsdomäne des Poly-Ig-Rezeptors, wobei dem Stabilisierungsprotein im wesentlichen jegliche C-terminalen Transmembran- und intrazellulären Poly-Ig-Rezeptordomänen fehlen,
wobei der Komplex gekennzeichnet ist durch proteolytische Stabilität im Vergleich zum nicht-komplexiertem Poly-Ig und wobei der Komplex weiterhin gekennzeichnet ist durch die spezifischen Bindungs- und immunprotektiven Vernetzungseigenschaften von Poly-Ig.

3. Im wesentlichen isolierter Komplex nach Anspruch 2, worin das Stabilisierungsprotein ein rekombinantes Protein ist.

4. Im wesentlichen isolierter Komplex nach Anspruch 1, 2 oder 3, worin das Poly-Ig und das Stabilisierungsprotein durch Disulfid-Bindungen kovalent verbunden sind.

5. Im wesentlichen isolierter Komplex nach Anspruch 1 oder Anspruch 2, worin das Poly-Ig ein Poly-Antikörper der IgA-Klasse ist.

6. Im wesentlichen isolierter Komplex nach Anspruch 1 oder Anspruch 2, worin das Stabilisierungsprotein die Domäne I und mindestens eine der Domänen IV und V des Poly-Ig-Rezeptors umfaßt und der Poly-Ig-Stabilisator so verkürzt ist, daß die Domäne VI im wesentlichen fehlt.

7. Im wesentlichen isolierter Komplex nach Anspruch 6, worin das Stabilisierungsprotein weiterhin dadurch gekennzeichnet ist, daß die Domäne I einen Abstand von der Domäne IV oder V besitzt, der einer Sequenz von ungefähr 100 bis 500 Aminosäuren entspricht.

8. Komplex nach Anspruch 1 oder Anspruch 2, umfassend einen monoklonalen IgA-Antikörper, welcher spezifisch für ein Antigen ist, ausgewählt aus der Gruppe, bestehend aus Antigenen von Pseudomonas aeruginosa, Hämophilus influenzae, Vibrio cholerae, Bordetella pertussis, Corynebacterium diphtheriae, Escherichia coli, Salmonella thyphi und typhimurium, Clostridium perfringens und anderen enterischen clostridiae, Shigella dysenteriae, Shigella flexnerii, Neisseria gonorrhoeae, Trichomonas, Entamoeba histolytica, Giardia lamblia, Streptococcus, Respiratorischen Syncytial-Virus, Rotavirus, Reovirus, humanem Immundefizienzvirus, humanem T-Zell-lymphotropem Virus, Typen I und II, Poliovirus, Rhinovirus, Influenzavirus, Herpesvirus, humanem Papillomavirus; Pneumocystis; Hefe; und Spermatozoen-Antigenen.

9. Rekombinantes Poly-Ig-Stabilisierungsprotein, umfassend eine Poly-Ig-Bindungsdomäne des Poly-Ig-Rezeptors, wobei dem Stabilisierungsprotein im wesentlichen jegliche C-terminalen Transmembran- und intrazellulären Poly-Ig-Rezeptordomänen fehlen und wobei das Stabilisierungsprotein das Merkmal aufweist, einen Komplex mit Poly-Ig zu bilden, der proteolytisch stabil ist und die immunprotektiven Vernetzungseigenschaften von Poly-Ig besitzt.

10. Verfahren zum Herstellen des Komplexes nach Anspruch 4, umfassend:
a) Bereitstellen einer Zelle, die so manipuliert ist, daß sie das Stabilisierungsprotein herstellt, wobei die Zelle auch dazu fähig ist, monoklonales Poly-Ig herzustellen, das spezifisch für das ausgewählte Antigen oder die Antigenfamilie ist;
b) Kultivieren der Zelle, so daß der Komplex hergestellt wird; und
c) Gewinnen des Komplexes.

11. Verfahren zum Herstellen des Komplexes nach Anspruch 2, worin das Poly-Ig und der Stabilisator über DisulfidBindungen kovalent verbunden sind, umfassend:
Bereitstellen haftabhängiger Epithelzellen, die so manipuliert sind, daß sie den Poly-Ig-Rezeptor herstellen,
Kultivieren der Epithelzellen unter Bedingungen, die eine einzellige Schicht der Zellen auf einem porösen Träger bilden, wobei der poröse Träger und die einzellige Schicht das Trennen eines ersten Mediums auf der basalen Seite der einzelligen Schicht von einem zweiten Medium auf der apikalen Seite der einzelligen Schicht erlaubt,
Einführen von monoklonalem IgA auf der basalen Seite der einzelligen Schicht,
Gewinnen des Komplexes von der apikalen Seite der einzelligen Schicht.

12. Verfahren nach Anspruch 11, worin das erste Medium ein Nährmedium ist, das geeignet zum Fördern des Wachstums der Epithelzellen ist.

13. Verfahren nach Anspruch 11, worin das zweite Medium im wesentlichen frei von Proteinen ist, die mit der Gewinnung des Komplexes interferieren.

14. Verfahren nach Anspruch 11, worin das monoklonale IgA in das erste Medium durch Co-Kultivieren von Hybridomazellen in dem ersten Medium eingebracht wird.

15. Verfahren nach Anspruch 11, worin das monoklonale IgA zuerst in einer Hybridomakultur hergestellt, dann von der Kultur abgetrennt und danach in das erste Medium eingebracht wird.

16. Verfahren zum Durchmustern von monoklonalem Poly-Ig, um den Schutz gegen ein Pathogen oder gegen Schwangerschaft in vivo zu stimmen, wobei das Verfahren die folgenden Schritte umfaßt:
a) Einbringen des monoklonalen Poly-Ig in den Kreislauf eines Säugers, der für das Pathogen empfänglich ist;
b) Testen der Schleimhautabsonderungen des Säugers auf Poly-Ig;
c) Applizieren (challenge) der Pathogene oder Spermatozoen auf eine Schleimhautoberfläche des Säugers;
d) Bestimmen des Schutzes gegen die Pathogen- oder Spermatozoenapplikation.

17. Verfahren nach Anspruch 16, worin das monoklonale Poly-Ig in den Säuger durch Injektion oder Infusion in den Kreislauf eingebracht wird.

18. Verfahren nach Anspruch 16, worin das monoklonale Poly-Ig in den Säuger durch subkutanes Implantieren eines Tumors oder einer tumorbildenden Zelle, welche(r) das monoklonale Poly-Ig herstellt, eingebracht wird.

19. Zusammensetzung, umfassend einen Komplex nach einem der Ansprüche 1 bis 3 für eine therapeutische Verabreichung an eine Schleimhautoberfläche.

20. Zusammensetzung nach Anspruch 19 für eine orale, nasale, vaginale, rektale therapeutische Verabreichung des Komplexes oder eine therapeutische Verabreichung in das Auge oder in das Mittelohr.

21. Zusammensetzung nach Anspruch 19, dadurch gekennzeichnet, daß die Zusammensetzung eine Schleimhautabsonderung eines ersten Säugers, umfassend den Komplex, ist, wobei die Schleimhautabsonderung gedacht ist für einen zweiten, zu schützenden Säuger.

22. Zusammensetzung nach Anspruch 21, dadurch gekennzeichnet, daß die Zusammensetzung Milch des ersten Säugers für eine orale Verabreichung des Komplexes an einen zweiten Säuger ist.

23. Zusammensetzung nach Anspruch 21 oder 22, erhältlich durch
a) Verabreichung einer Poly-Ig produzierenden, immortalisierten Zelle an den ersten Säuger, so daß das Poly-Ig des Komplexes im Kreislauf des ersten Säugers hergestellt wird, und
b) Sammeln der den Komplex umfassenden Schleimhautabsonderung von dem ersten Säuger, wobei die Schleimhautabsonderung für die Verabreichung des Komplexes an einen zweiten Säuger ist.

## Revendications

1. Complexe sensiblement isolé comprenant
a) une poly-Ig spécifique d'un antigène sélectionné ou d'une famille d'antigènes sélectionnée ; et
b) une protéine recombinante stabilisatrice comprenant un domaine de liaison à la poly-Ig d'un récepteur de poly-Ig, ladite protéine stabilisatrice étant sensiblement dépourvue de tout domaine récepteur de poly-Ig intracellulaire et transmembranaire C-terminal ;
ledit complexe étant caractérisé par une stabilité protéolytique comparé à une poly-Ig non complexée, ledit complexe étant en outre caractérisé par les propriétés de liaison spécifique et de réticulation immunoprotectrice des poly-Ig.

2. Complexe sensiblement isolé comprenant
a) une poly-Ig monoclonale spécifique d'un antigène sélectionné ou d'une famille d'antigènes sélectionnée ; et
b) une protéine stabilisatrice comprenant un domaine de liaison à la poly-Ig du récepteur de poly-Ig, ladite protéine stabilisatrice étant sensiblement dépourvue de tout domaine récepteur de poly-Ig intracellulaire et transmembranaire C-terminal ;
ledit complexe étant caractérisé par une stabilité protéolytique comparé à une poly-Ig non complexée, ledit complexe étant en outre caractérisé par les propriétés de liaison spécifique et de réticulation immunoprotectrice des poly-Ig.

3. Complexe sensiblement isolé de la revendication 2 dans lequel ladite protéine stabilisatrice est une protéine recombinante.

4. Complexe sensiblement isolé de la revendication 1, 2 ou 3 dans lequel ladite poly-Ig et ladite protéine stabilisatrice sont reliées de manière covalente par des liaisons disulfure.

5. Complexe sensiblement isolé de la revendication 1 ou de la revendication 2 dans lequel ladite poly-Ig est un anticorps de la classe des poly-IgA.

6. Complexe sensiblement isolé de la revendication 1 ou de la revendication 2 dans lequel ladite protéine stabilisatrice comprend le domaine I et au moins l'un des domaines IV et V du récepteur de poly-Ig et ladite protéine stabilisatrice de poly-Ig est tronquée de manière à déléter pour l'essentiel le domaine VI.

7. Complexe sensiblement isolé de la revendication 6 dans lequel ladite protéine stabilisatrice est en outre caractérisée en ce que ledit domaine I est éloigné dudit domaine IV ou V d'une distance correspondant à une séquence d'environ 100 à 500 acides aminés.

8. Complexe de la revendication 1 ou de la revendication 2, comprenant un anticorps monoclonal de type IgA spécifique d'un antigène choisi dans le groupe constitué par les antigènes de Pseudomonas aeruginosa, d'Hemophilus influenzae, de Vibrio cholerae, de Bordetella pertussis, de Corynebacterium diphtheriae, d'Escherichia coli, de Salmonella typhi et typhimurium, de Clostridium perfringens et d'autres clostridiae entériques, de Shigella dysenteriae, de Shigella flexnerii, de Neisseria gonorrheae, de Trichomonas, d'Entameba histolytica, de Giardia lamblia, de Streptococcus, du virus syncitial respiratoire, d'un rotavirus, d'un réovirus, du Virus de l'Immunodéficience Humaine, du Virus Lymphotrophe de cellules T humaines, du poliovirus de types I et II, du Rhinovirus, du virus de la grippe, des virus de l'herpes, du virus du papillome humain ; de Pneumocystis ; de levure ; et d'antigènes de spermatozoïdes.

9. Protéine stabilisatrice recombinante de poly-Ig comprenant un domaine de liaison à la poly-Ig du récepteur de poly-Ig, ladite protéine stabilisatrice étant sensiblement dépourvue de tout domaine récepteur de poly-Ig intracellulaire et transmembranaire C-terminal, ladite protéine stabilisatrice présentant la caractéristique de former un complexe avec la poly-Ig qui est stable au regard de la protéolyse et possède les propriétés de réticulation immunoprotectrice des poly-Ig.

10. Procédé de préparation du complexe de la revendication 4 comprenant les étapes consistant à :
a) se procurer une cellule manipulée afin qu'elle produise ladite protéine stabilisatrice, ladite cellule étant également capable de produire une poly-Ig monoclonale spécifique dudit antigène sélectionné ou de ladite famille d'antigènes sélectionnée ;
b) cultiver ladite cellule pour produire ledit complexe ; et
c) récupérer ledit complexe.

11. Procédé de préparation du complexe de la revendication 2 dans lequel ladite poly-Ig et ladite protéine stabilisatrice sont reliés de manière covalente par des liaisons disulfure, comprenant les étapes consistant à :
se procurer des cellules épithéliales ancragedépendantes manipulées afin de produire un récepteur de poly-Ig,
cultiver lesdites cellules épithéliales dans des conditions formant une monocouche desdites cellules sur un support poreux, lesdits support et monocouche formant une séparation entre un premier milieu du côté de la base de la monocouche et un deuxième milieu du côté du sommet de la monocouche,
introduire une IgA monoclonale du côté de la base de la monocouche,
récupérer ledit complexe du côté du sommet de la monocouche.

12. Procédé de la revendication 11, dans lequel ledit premier milieu est un milieu nutritif qui convient pour aider à la croissance desdites cellules épithéliales.

13. Procédé de la revendication 11 dans lequel ledit deuxième milieu est sensiblement dépourvu de protéines interférant avec la récupération dudit complexe.

14. Procédé de la revendication 11 dans lequel ladite IgA monoclonale est introduite dans ledit premier milieu par co-culture de cellules d'hybridomes dans ledit premier milieu.

15. Procédé de la revendication 11 dans lequel ladite IgA monoclonale est d'abord produite dans une culture d'hybridomes, puis séparée de ladite culture et ensuite introduite dans ledit premier milieu.

16. Procédé de criblage in vivo de poly-Ig monoclonale pour déterminer la protection contre un pathogène ou la grossesse, ledit procédé comprenant les étapes consistant à
a) introduire ladite poly-Ig monoclonale dans la circulation d'un mammifère sensible au pathogène ;
b) tester les poly-Ig des sécrétions des muqueuses dudit mammifère ;
c) éprouver une surface de muqueuse dudit mammifère avec lesdits pathogènes ou spermatozoïdes ;
d) déterminer la protection contre l'agression du pathogène ou des spermatozoïdes.

17. Procédé de la revendication 16 dans lequel la poly-Ig monoclonale est introduite dans le mammifère par injection ou infusion dans la circulation.

18. Procédé de la revendication 16 dans lequel la poly-Ig monoclonale est introduite dans le mammifère par implantation sous-cutanée d'une tumeur ou d'une cellule formant tumeur qui produit la poly-Ig monoclonale.

19. Composition comprenant un complexe de l'une quelconque des revendications 1-3 pour administration thérapeutique à une surface muqueuse.

20. Composition de la revendication 19 pour administration thérapeutique du complexe par voie orale, nasale, vaginale, rectale, oculaire, ou par l'oreille moyenne.

21. Composition de la revendication 19 caractérisée en ce qu'il s'agit d'une sécrétion muqueuse d'un premier mammifère comprenant ledit complexe, ladite sécrétion muqueuse étant destinée à l'administration à un second mammifère devant être protégé.

22. Composition de la revendication 21 caractérisée en ce qu'elle est le lait dudit premier mammifère, pour administration par voie orale dudit complexe au deuxième mammifère.

23. Composition de la revendication 21 ou 22 pouvant être obtenue par
a) administration au premier mammifère d'une cellule immortalisée produisant la poly-Ig afin de produire ladite poly-Ig dudit complexe dans la circulation du premier mammifère, et
b) récupération de la sécrétion muqueuse du premier mammifère comprenant ledit complexe, la sécrétion muqueuse étant pour administration dudit complexe au second mammifère.
